# EUROPEAN PATENT SPECIFICATION

(11) **EP 3 441 393 B1**
(45) Date of publication and mention of the grant of the patent: **18.03.2020**
(21) Application number: 17306053.4
(22) Date of filing: 07.08.2017
(51) Int. Cl.: C07D 493/08, C07C 315/04, C07C 317/14

(54) **NEW CYCLOADDUCT PRECURSORS OF DIHALODIPHENYLSULFONES AND PREPARATIONS THEREOF**
NEUE CYCLOADDUKTVORLÄUFER VON DIHALODIPHENYLSULFONEN UND ZUBEREITUNGEN DAVON
NOUVEAUX PRÉCURSEURS CYCLOADDUITS DE DIHALOGÉNODIPHÉNYLSULFONES ET LEURS PRÉPARATIONS

(43) Date of publication of application: 13.02.2019
(73) Proprietor: RHODIA OPERATIONS, 75009 Paris (FR)
(72) Inventor: MULLER, Eric, 69003 Lyon (FR)
(74) Representative: Delenne, Marc

(56) References cited:
- EP-A2- 0 381 045
- WO-A1-2016/201039
- US-A- 4 871 876
- YUR'EV ET AL.: "Furan series.VIII. Tetramethylfuran in the Diels-Alder reaction", ZHURNAL OBSHCHEI KHIMII, vol. 30, 1960, pages 3214-3217, XP009501540, & DATABASE REGISTRY [Online] CHEMICAL ABSTRACTS SERVICE, COLUMBUS, OHIO, US; 3 December 2009 (2009-12-03), Database accession no. 1195719-32-4

## Description

### TECHNICAL FIELD

The present invention pertains, as new and useful chemical compounds, to specific di-(halo-oxanorbornene)sulfones, obtainable by cycloaddition between a divinylsulfone and a halofuran (Diels-Alder reaction) and to their use as precursors for the preparation of dihalodiphenylsulfones.

### BACKGROUND ART

Dihalodiphenylsulfones, in particular 4,4'-dichlorodiphenylsulfones (DCDPS), are valuable chemical compounds which are known to the art. They may be used, for instance, as monomers in the production of polarylethersulfones, which are a family of thermoplastics known as engineering plastics for use in high temperature applications, for example in the fields of medical equipment, appliances, automobiles parts and electrical equipment. DCDPS have also been used as pesticide and in reactive dyes in the textile industry. For certain applications, 4,4'-dichlorodiphenyl sulfones that are substantially free of the 2,4' and 3,4'-dichlorodiphenylsulfone isomers are necessary.

Heretofore, several processes for the manufacture of di-(halophenyl)sulfones, in particular 4,4'-dichlorodiphenylsulfone, have been proposed and which involve reactants made from non-renewable resources.

US 2971985 discloses a process for the preparation of 4,4'-dichlorodiphenylsulfone which comprises reacting an equimolar mixture of dimethylpyrosulfate and sulfur trioxide with chlorobenzene at temperatures below 100 °C and recovering 4,4'-dichlorodiphenylsulfone.

In US 3355497, a 4,4'-dichlorodiphenylsulfone is prepared by reacting a mixture of one mole of dimethyl pyrosulfate and two to three moles of sulfur trioxide with at least one mole of chlorobenzene over the number of moles of sulfur trioxide at a temperature of from room temperature to 100 °C.

GB 1572916 discloses a process for producing 4,4'-dichlorodiphenylsulfone comprising the steps of: a) reacting monochlorobenzene with sulfur trioxide to form p-chlorobenzene sulfonic acid; b) reacting the product of step a) with thionyl chloride in the presence of a catalytic amount of N,N-dimethylformamide at a temperature sufficient to form p-chlorobenzene sulfonyl chloride; and c) reacting the product of step b) with monochlorobenzene in the presence of a catalytic amount of ferric chloride at a temperature sufficient to produce 4,4'-dichlorodiphenyl sulfone.

US 4871876 discloses a process for the preparation of 4,4'-dichlorodiphenyl sulfone by heating a mixture of (a) chlorobenzene, (b) chlorosulfonic acid or sulfur trioxide, and (c) thionyl chloride or phosgene to temperatures up to 220 °C, more than the stoichiometric amount (based on the amount of chlorobenzene) of chlorosulfonic acid or sulfur trioxide and less than the stoichiometric amount of thionyl chloride or phosgene being employed.

At last, EP 0381045 discloses a process for the preparation of bis(4-chlorophenyl) sulfone by reacting chlorobenzene at 150 to 280 °C and 1.3 to 6 bars with chlorobenzenesulfonic acid in the presence of polyphosphoric acid or a mixture of phosphorus pentoxide and a non-aromatic sulfonic acid.

There remains a need for an efficient process for the preparation of dihalodiphenylsulfones. There is also need for a process for the preparation of dihalodiphenylsulfones involving renewable starting materials

In this context, the present invention aims at providing a new and efficient process for the preparation of dihalodiphenylsulfones. Another objective of the present invention is to provide a process for the preparation of dihalodiphenylsulfones involving renewable starting materials.

### SUMMARY OF THE INVENTION

The present invention therefore relates to a compound of formula (I) 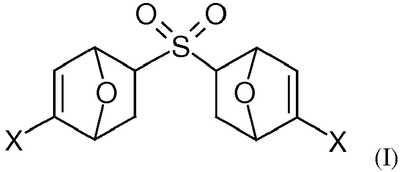 wherein X represents a halogen atom selected from the group consisting of fluorine, chlorine, bromine and iodine.

Preferably, the halogen atom is chlorine or bromine, more preferably chlorine.

Another object of the invention relates to a process for the preparation of a compound of formula (I) as defined above, comprising reacting divinylsulfone with a halofuran of formula (II) 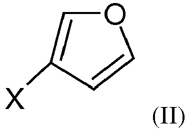 wherein X is as defined above,
and optionally further isolating compound of formula (I).

Preferably, the halofuran is 3-chlorofuran or 3-bromofuran.

According to an embodiment, the molar ratio of the halofuran of formula (II) to divinylsulfone is of at least 2/1.

The present invention further relates to the use of a compound of formula (I) as defined above, for the preparation of a 4,4'-dihalodiphenylsulfone of formula (III) 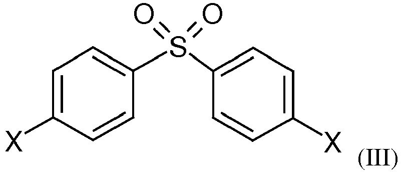 wherein X is as defined above.

Another object of the invention relates to a process for the preparation of a 4,4'-dihalodiphenylsulfone of formula (III) 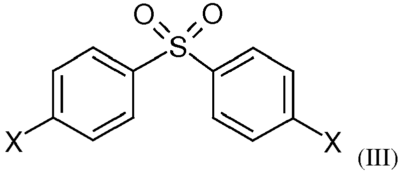 wherein X represents a halogen atom selected from the group consisting of fluorine, chlorine, bromine and iodine,
comprising carrying out the dehydration/aromatization of a compound of formula (I) 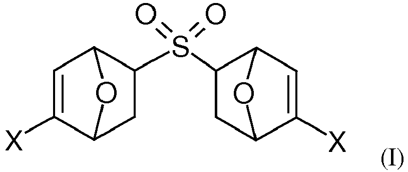 wherein X is as defined above.

According to an embodiment, the dehydration/aromatization is carried out in the presence of an alkali hydroxide, preferably sodium hydroxide or potassium hydroxide.

According to an embodiment, the dehydration/aromatization is carried out in the presence of a solvent, preferably DMSO.

Another object of the invention relates to a process for the preparation of a 4,4'-dihalodiphenylsulfone of formula (III) 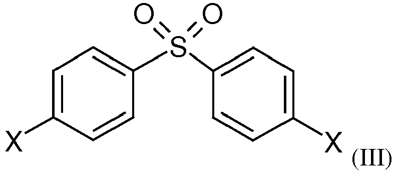 wherein X represents a halogen atom selected from the group consisting of fluorine, chlorine, bromine and iodine, comprising:
a) providing a compound of formula (I) 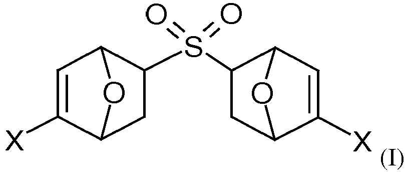 wherein X is as defined above,
b) carrying out the dehydration/aromatization of said compound of formula (I),
c) optionally further isolating compound of formula (III).

According to an embodiment of the invention, compound of formula (I) is prepared by reacting divinylsulfone with a halofuran of formula (II) 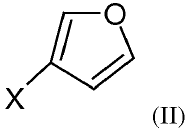 wherein X is as defined above.

As already mentioned, one of the advantages of the invention is to provide a process for the preparation of 4,4'-dihalodiphenylsulfones involving renewable starting materials.

Moreover, it has been surprisingly found that the process for the preparation of compound of formula (I) according to the invention allows satisfactory conversion and selectivity.

### DETAILED DESCRIPTION OF THE INVENTION

As used herein, the terms "a", "an", or "the" means "one or more" or "at least one" unless otherwise stated. Throughout the present disclosure, various publications may be cited and/or incorporated by reference. Should the meaning of any language in such publications conflict with the meaning of the language of the present disclosure, the meaning of the language of the present disclosure shall take precedence, unless otherwise indicated.

The present invention is directed to a compound of formula (I) 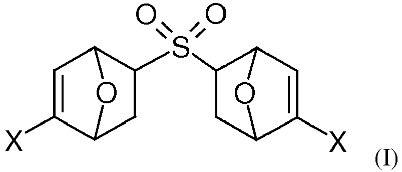 wherein X represents a halogen atom selected from the group consisting of fluorine, chlorine, bromine and iodine.

In a preferred embodiment of the invention, the halogen atom is bromine and chlorine, more preferably chorine.

Compound of formula (I) may be prepared from divinylsulfone and a halofuran of formula (II) 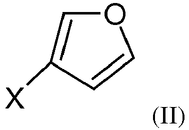 wherein X is as defined above.

The halofuran of formula (II) can be obtained from furan according to processes known to the person skilled in the art. Document US 2,773,882 describes such a process.

Furan may be prepared by decarbonylation of furfural which is obtained from a renewable resource. Suitable renewable resources as well as suitable methods for their conversion into furfural are known to the person skilled in the art.

Alternatively, the halofuran of formula (II) may be prepared by any other conventional chemical reactions, or may be also commercially available products.

Divinylsulfone can be obtained from ethylene oxide via thiodiglycol as described in document US 2,278,090, or from acetylene via divinylsulfide as described in Sulfur Reports, Volume 3(9), pp. 323-400 (1984) and in Chinese Journal of Catalysis, 36, pp. 1342-1349 (2015). Both ethylene oxide and acetylene may be obtained from renewable resources.

It has been surprisingly found that a Diels-Alder reaction efficiently occurs between the halofuran of formula (II) and divinylsulfone resulting in the formation of a double cycloadduct of formula (I) as defined above. Moreover, it has been surprisingly found that the Diels-Alder reaction between the halofuran of formula (II) and divinylsulfone leads to the formation of compound of formula (I) with a good conversion, a low amount of by-products and a good selectivity (i.e. para/para), even without the use of a catalyst.

The present invention therefore also provides a process for the production of a compound of formula (I), comprising reacting divinylsulfone with a halofuran of formula (II).

The Diels-Alder reaction between the halofuran of formula (II) and divinylsulfone leads to the formation of two main regioisomers represented below, one of which being the compound of formula (I):

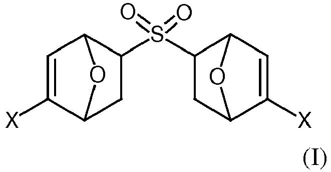

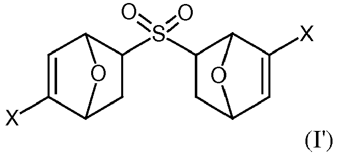

Other isomers might be also obtained in minor amounts, in particular the compound of formula (I")

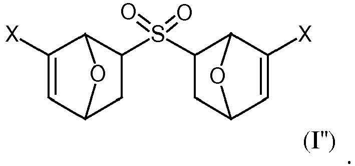

Regioisomers of formula (I), (I') and (I") can be present as endo- or exo- isomer. In particular, each cycle of the regioisomers can be either in exo configuration or endo configuration, also these possible isomers are included within the scope of the present invention.

According to an embodiment of the invention, the molar ratio of compound of formula (I) to compound of formula (I') is of at least 50/50, preferably at least 70/30, more preferably at least 80/20.

According to an embodiment of the invention, the conversion rate of the Diels-Alder reaction is of at least 50%, preferably at least 70%, more preferably at least 90%.

The Diels-Alder reaction between the halofuran of formula (II) and divinylsulfone can be carried out under usual Diels-Alder conditions known to the person skilled in the art.

The Diels-Alder reaction may be carried out with or without a solvent. Suitable solvents may be selected from pyridine, tertiary amines such as triethylamine and diisopropylethylamine, chloroform, dichloromethane, diethyl ether, ethanol, methanol, perfluorinated alkanes such as perfluorohexane, toluene, water and ionic liquids such as 1-butyl-3-methylimidazolium tetrafluoroborate and 1-butyl-3-methylimidazolium hexafluorophosphate, alone or in a mixture. Preferred solvents are pyridine and tertiary amines, such as triethylamine.

The Applicant has surprisingly noticed that certain basic additives such as pyridine and tertiary amines, such as triethylamine, stabilize the reactants which allows an excess of the halofuran of formula (II) to be used in order to increase the conversion of the Diels-Alder reaction. The excess of the halofuran used may be recovered once the reaction is completed.

The reaction may be carried out at any suitable temperature, for example from about 10 to about 120 °C, preferably from about 20 to about 100 °C, more preferably from about 30 to about 80 °C, for a time sufficient to convert the starting compounds into the desired Diels-Alder adduct, such as about 10 minutes to about 6 days, preferably about 3 hours to about 4 days, more preferably about 12 hours to about 2 days.

The reaction can be carried out at ambient pressure or under increased pressure. In a preferred embodiment, the reaction is carried out at ambient pressure, such as about 1 bar, or at a pressure of up to 10 bars, preferably up to 5 bars, more preferably up to 2 bars.

The Diels-Alder reaction may be conducted with or without a catalyst. The use of catalysts can improve kinetics and selectivity of the Diels-Alder reaction. Known Diels-Alder catalysts may be used and may include Lewis acids, such as aluminium chloride, ethylaluminium dichloride, diethylaluminium chloride, trimethyl aluminium, bismuth (III) chloride, bismuth (III) trifluoromethanesulfonate, boron trifluoride, boron triacetate, cerium (III) chloride, copper (I) trifluoromethanesulfonate, copper (II) chloride, hafnium (IV) chloride, iron (II) chloride, iron (II) acetate, iron (III) chloride, iron (III) acetate, lithium perchlorate, lithium trifluoromethanesulfonate, magnesium bromide, magnesium iodide, scandium (III) trifluoromethanesulfonate, tin (IV) chloride, titanium (IV) chloride, titanium (IV) isopropoxide, N-trimethylsilyI-bis(trifluoromethanesulfonyl)imide, trimethylsilyl trifluoromethanesulfonate, ytterbium (III) trifluoromethanesulfonate, zinc chloride, zinc bromide, zinc iodide, zinc acetate and zirconium (IV) chloride and mixtures thereof.

Bronsted acids, such as inorganic mineral acids, e.g. sulphuric acid, phosphoric acid, nitric acid, hydrobromic acid or hydrochloric acid, and organic acids such as methane sulphonic acid, p-toluenesulphonic acid or carboxylic acids. Alternatively, activated carbon, silica, alumina, silica-alumina, zirconia and zeolites may be used as such or as support for a catalytically active metal or metal compound. Suitable metals or metal compounds include alkali metals, alkaline earth metals, transition metals, noble metals, rare earth metals. The catalysts can be acidic, e.g. by treating supports with phosphoric acid, or by ion exchange of zeolites to render them into their acidic form. Examples of solid catalysts include amorphous silica-alumina, zeolites, preferably zeolites in their H-form, and acidic ion exchange resins. Other suitable catalysts that are liquids or that may be dissolved in the appropriate solvent to yield a homogeneous catalyst environment, include organic and inorganic acids, such as alkane carboxylic acid, arene carboxylic acid, sulphuric acid, phosphoric acid, hydrochloric acid, hydrobromic acid and nitric acid.

According to an embodiment of the invention, the molar ratio of the halofuran of formula (II) to divinylsulfone is close to stoichiometry (namely 2/1), or above.

The Applicant has found that a molar ratio of the halofuran of formula (II) to divinylsulfone greater than 2/1 allows for the displacement of the Diels-Alder reaction towards the formation of the cycloadduct of formula (I).

The process according to the invention may comprise a further step of isolation of compound of formula (I). Suitable methods of isolation and purification are known to the person skilled in the art. For example, chromatography techniques, such as liquid chromatography, and recrystallization may be particularly efficient in the process according to the invention.

The present invention also relates to a process for the preparation of a 4,4'-dihalodiphenylsulfone of formula (III) 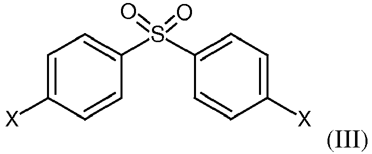 wherein X represents a halogen atom selected from the group consisting of fluorine, chlorine, bromine and iodine, comprising carrying out the dehydration/aromatization of the compound of formula (I) as defined above. Preferably, X is selected from chlorine and bromine, more preferably chlorine.

The dehydration/aromatization may be represented by the following scheme:

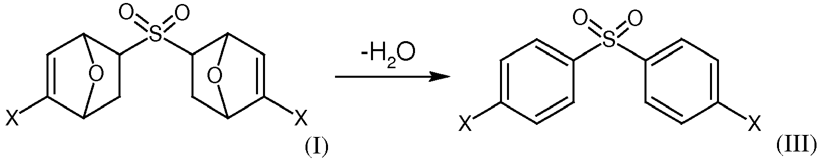

The reaction conditions for aromatization of the compound of formula (I) are known to a person skilled in the art.

According to an embodiment of the invention, the aromatization reaction of the compound of formula (I) requires basic reaction conditions, for example in the presence of a methoxide compound or hydroxide compound. Suitable methoxides may be selected from alkali methoxides, such as sodium methoxide and potassium methoxide, preferably sodium methoxide. Suitable hydroxides may be selected from alkali hydroxides, such as sodium hydroxide, potassium hydroxide, lithium hydroxide, cesium hydroxide, preferably sodium hydroxide and potassium hydroxide, more preferably sodium hydroxide.

Suitable solvents for the aromatization reaction may be for example DMSO.

The aromatization reaction may be carried out at any suitable temperature, for example from about 10 to about 120 °C, preferably from about 20 to about 100 °C, more preferably from about 40 to about 80 °C.

Another object of the invention relates to a process for producing a 4,4'-dihalodiphenylsulfone of formula (III) 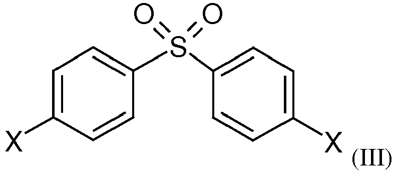 wherein X represents a halogen atom selected from the group consisting of fluorine, chlorine, bromine and iodine, comprising:
a) providing a compound of formula (I) as defined above,
b) carrying out the dehydration/aromatization of said compound of formula (I).

According to an embodiment of the invention, the compound of formula (I) is obtained by a Diels-Alder reaction between divinylsulfone and the above-described halofuran of formula (II) according to the process previously detailed.

Once the Diels-Alder reaction is completed, isolation of composition of formula (I) may be carried out before step b).

Alternatively, the mixture obtained at the end of the Diels-Alder reaction and comprising regioisomers of formula (I), (I') and (I") can be directly used in the subsequent step of dehydration/aromatization. In this case, a mixture comprising the two following regioisomers is obtained after completing the aromatization:

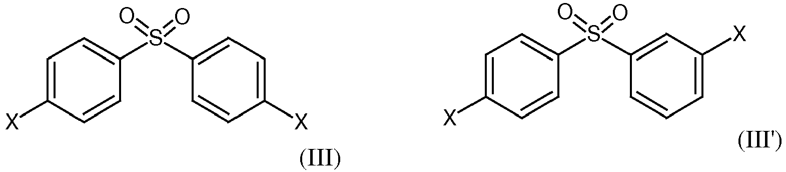

According to an embodiment of the invention, 4,4'-dihalodiphenylsulfone of formula (III) is further isolated by separation methods known to the person skilled in the art. For example, recrystallization may be carried out according to the method described in document US 4,873,372.

In accordance with the present invention, the 4,4'-dihalodiphenylsulfone can be used in a variety of applications.

Firstly, the 4,4'-dihalodiphenylsulfone can be used for the manufacture of a polymer, such as a polyarylethersulfone.

As herein used, the term "polyarylethersulfone" is intended to denote any polymer comprising repeating units comprising at least one arylene group, at least one ether group and at least one sulfonyl ( 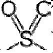 ) group. The polyarylethersulfone manufactured according to the present invention comprises typically repeat units comprising 4,4'-diphenysulfone moieties of formula (IV)

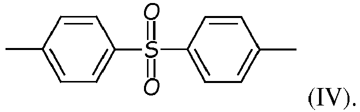

In an embodiment, the invention concerns a process for the manufacture of a polyarylethersulfone comprising the steps of :
i) preparing a 4,4'-dihalodiphenylsulfone of formula (III) as defined above by carrying out the dehydration/aromatization of a compound of formula (I) as defined above,
ii) optionally, isolating the 4,4'-dihalodiphenylsulfone,
iii) forming a reaction mixture comprising the 4,4'-dihalodiphenylsulfone and at least one of M¹ and M², wherein M¹ is at least one dibasic salt of a dihydroxy aromatic compound and M² consists of at least one dihydroxy aromatic compound and at least one basic compound,
iv) carrying out a polycondensation reaction of the reaction mixture.

In another embodiment, the invention concerns a process for the manufacture of a polyarylethersulfone comprising the steps of :
i) preparing a compound of formula (I) as defined above by reacting divinylsulfone with a halofuran of formula (II) as above defined,
ii) preparing a 4,4'-dihalodiphenylsulfone of formula (III) as defined above by carrying out the dehydration/aromatization of the compound of formula (I),
iii) optionally, isolating 4,4'-dihalodiphenylsulfone,
iv) forming a reaction mixture comprising the 4,4'-dihalodiphenylsulfone and at least one of M¹ and M², wherein M¹ is at least one dibasic salt of a dihydroxy aromatic compound and M² consists of at least one dihydroxy aromatic compound and at least one basic compound,
v) carrying out a polycondensation reaction of the reaction mixture.

In the above two processes for the manufacture of a polyarylethersulfone, it is preferred to isolate the 4,4'-dihalodiphenylsulfone before forming the reaction mixture and carrying out the polycondensation reaction.

The dihydroxy aromatic compound is advantageously chosen from bisphenol A, bisphenol S, 4'4'-oxydiphenol, 4,4'-dihydroxybiphenyl, p-hydroquinone and mixtures thereof.

The basic compound is advantageously an alkali metal compound, in particular a sodium or potassium derivative. Besides, it is often a bicarbonate, a carbonate or a hydroxide. The basic compound is preferably an alkali metal carbonate or an alkali metal hydroxide, more preferably an alkali metal carbonate. Non limitative examples of suitable basic compounds are KOH and K₂CO₃; mixtures of K₂CO₃ and Na₂CO₃ are also suitable. The basic compound has advantageously an average particle size of up to about 100µm. The basic compound is preferably in its anhydrous form. Excellent results are obtained when using anhydrous potassium carbonate having an average particle size from about 10 µm to about 60 µm.

The dibasic salt of a dihydroxy aromatic compound is advantageously a dibasic alkali metal salt, in particular a disodium or dipotassium salt.

Often, the reaction mixture is free of monomer other than the 4,4'-dihalodiphenylsulfone, the dihydroxyaromatic compound and the dibasic salt of a dihydroxy aromatic compound. Yet, sometimes (typically, when a specialty polyarylethersulfone is intended to be manufactured), the reaction mixture further comprises one or more other additional monomer(s). Among such additional possible monomer(s), it can be cited aromatic dihalosulfones other than 4,4'-dihalodiphenylsulfones [like 4,4'-bis-(4-chlorophenyl sulfonyl)biphenyl)], as well as aromatic compounds comprising one and only one halogen group and one and only one hydroxyl group (like 4-hydroxy-4'-chlorodiphenyl sulfone) and their monobasic salts homologues.

The 4,4'-dihalodiphenylsulfone and, as the case may be, the dibasic salt M¹ of a dihydroxy aromatic compound and/or the dihydroxy aromatic compound comprised in M², are generally used in a molar ratio from 0.90 to 1.10, preferably in substantially equimolar ratios (e.g. in molar ratios ranging from 0.98 to 1.02, or from 0.99 to 1.01).

When the reaction mixture comprises M², the basic compound, in particular the alkali metal compound, is generally used in an amount of from 0.50 to 1.50 mole, preferably from 1.00 to 1.10 mole, per mole of hydroxyl group of the dihydroxy aromatic compound. Still more preferably, a slight excess of the basic compound is used, meaning that the basic compound is for example used in an amount of from 1.01 to 1.10 mole or from 1.05 to 1.10 mole, per mole of hydroxyl group.

Advantageously, the reaction mixture further comprises a polar aprotic solvent (such as dimethylsulfoxide, dimethylformamide, N-methyl pyrolidinone or diphenylsulfone) or a solvent system comprising a polar aprotic solvent. An azeotrope-forming solvent such as chlorobenzene is sometimes used as cosolvent, in addition to the polar aprotic solvent.

Examples of carrying out polycondensation reactions of the 4,4'-dihalodiphenylsulfone with M¹ or M² are notably provided in GB 1 559 855, in US 4,108,837, in US 6,228,970 and in US 6,593,445, the whole content of which is herein incorporated by reference for all purposes.

The polyarylethersulfone manufactured by the above two processes comprises generally recurring units (R1) : 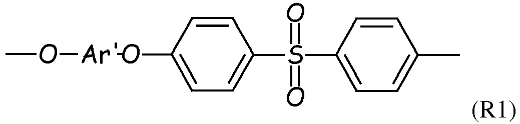 wherein Ar' is an aromatic divalent group, such as : 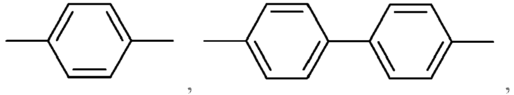 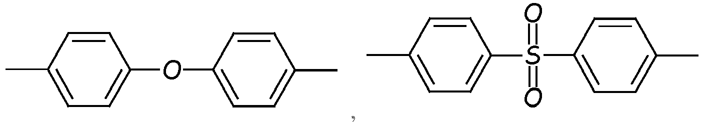 and 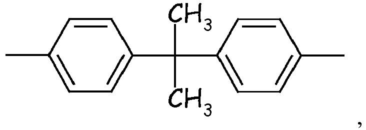 in a weight amount, based on the total weight of the recurring units of the polyarylethersulfone, that exceeds 50 %. Very often, the weight amount of recurring units (R1), based on the total weight of the recurring units of the polyarylethersulfone, exceeds 90 %. Often, the polyarylethersulfone comprises recurring units (R1) as sole recurring units, i.e. the polyarylethersulfone is a homopolymer.

Polyarylethersulfones of high industrial importance that can be manufactured by the above two processes include :
- polysulfones, also named bisphenol A polysulfones, i.e. homopolymers the recurring units of which are recurring units (R2) : 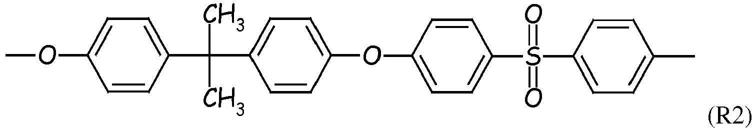
- polyethersulfones, i.e. homopolymers the recurring units of which are recurring units (R3) : 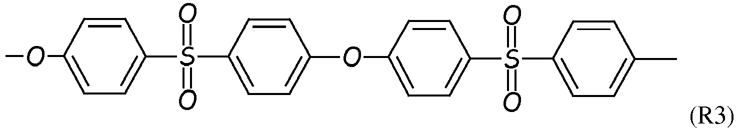 and
- polyphenylsulfones, i.e. homopolymers the recurring units of which are recurring units (R4) :

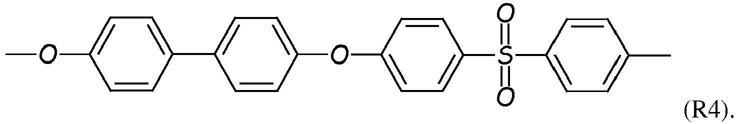

Polysulfones (PSU), polyethersulfones (PESU) and polyphenylsulfones (PPSU) are available from Solvay Specialty Polymers USA, L.L.C. respectively as as UDEL® PSU, VERADEL® PESU and RADEL® PPSU.

The 4,4'-dihalodiphenylsulfone can also be used for the manufacture of a polymer other than polyarylethersulfone, such as a modified polyphenylene. As herein used, the term "polyphenylene" is intended to denote any polymer comprising phenylene units, often p-phenylene units or a mixture of p-phenylene units and m-phenylene units. The modified polyphenylene according to the invention comprises typically 4,4'-diphenylsulfone moieities of formula (IV) as above depicted as repeat units, in addition to phenylene repeat units.

The polymer, especially the polyarylethersulfone, can in turn be solution processed or melt processed (for example extruded or injection molded), to form a shaped article like a membrane, a sterilization tray or case, a dental or surgical instrument, an aircraft interior, an airline catering trolley, a hot water fitting, a plumbing manifold, an electrical or electronic component, a faucet component, a fuel system component, a food service component or an orthopedic device, etc.

Secondly, the 4,4'-dihalodiphenylsulfone can be used for the manufacture of 4,4'-diaminodiphenylsulfone.

In this regard, the invention concerns a process for the manufacture of 4,4'-diaminodiphenylsulfone comprising the steps of:
i) preparing a 4,4'-dihalodiphenylsulfone of formula (III) as defined above by carrying out the dehydration/aromatization of a compound of formula (I) as defined above,
ii) optionally, isolating the 4,4'-dihalodiphenylsulfone,
iii) carrying out the amination reaction of the 4,4'-dihalodiphenylsulfone with an aminating agent, especially carrying out the ammonolysis reaction of the 4,4'-dihalodiphenylsulfone with ammonia.

It concerns also a process for the manufacture of 4,4'-diaminodiphenylsulfone comprising comprising the steps of:
i) preparing a compound of formula (I) as defined above by reacting divinylsulfone with a halofuran of formula (II) as above defined,
ii) preparing a 4,4'-dihalodiphenylsulfone of formula (III) as defined above by carrying out the dehydration/aromatization of the compound of formula (I),
iii) optionally, isolating 4,4'-dihalodiphenylsulfone,
iv) carrying out the amination reaction of the 4,4'-dihalodiphenylsulfone with an aminating agent, especially carrying out the ammonolysis reaction of the 4,4'-dihalodiphenylsulfone with ammonia.

In the above two processes for the manufacture of 4,4'-diaminodiphenylsulfone, it is preferred to isolate the 4,4'-dihalodiphenylsulfone before carrying out the amination reaction.

A first example of carrying out the ammonolysis reaction of the 4,4'-dihalodiphenylsulfone with ammonia is provided in J. Am. Chem. Soc., vol. 67, 11, pp. 1979-1986, "Derivatives of p,p'-Diaminodiphenyl Sulfone", the whole content of which is herein incorporated by reference for all purposes. Another example of carrying out the ammonolysis reaction of the 4,4'-dihalodiphenylsulfone with ammonia is provided in CN 103819372 A, the whole content of which is also herein incorporated by reference for all purposes.

The so-manufactured 4,4'-dihalodiphenylsulfone can in turn be used :
- as a curing agent, e.g. to cure an epoxy resin or an isocyanate prepolymer,
- as a therapeutic or animal bacterial infection therapeutic agent, notably for leprosy treatment or as an inhibitor of dermatitis herpetiformis,
- as a monomer for the manufacture of a polymer such as a semi-aromatic or wholly aromatic polyamide (typically by carrying out a polycondensation reaction of the 4,4'-diaminodiphenylsulfone with respectively an aliphatic or aromatic dicarboxylic acid compound) or a polyurea (typically by polymerizing the 4,4'-diaminodiphenylsulfone with a diisocyanate).

Still other possible uses of the 4,4'-dihalodiphenylsulfone prepared in accordance with the present invention include :
- its use as an additive in a reactive dye, typically in the textile industry,
- its use as a pesticide, and
- the synthesis of various other 4,4'-dichlorodiphenylsulfone derivatives, such as 4-chloro-4'-aminodiphenylsulfone, diphenylsulfone 4,4'-bisisocyanate, disodium 4,4'-bisbenzensulfonylaminodiphenylsulfone, 4,4'-bissuccinoylaminodiphenylsulfone and 4,4'-bisstearylaminodiphenylsulfone, the synthesis of all these derivatives and still other ones being notably described in above cited publication entitled "*Derivatives of p,p'-Diaminodiphenyl Sulfone*".

The present invention will now be illustrated by the following examples, which are not intended to be limiting.

### EXAMPLES

### 1) Preparation of 4,4'-dichlorodiphenylsulfone

### 1.1) Diels-Alder reaction of 3-chlorofuran with divinylsulfone

In a carousel tube fitted with a PTFE septum screw cap, divinylsulfone (0.712 g; 6.0 mmol) was weighted. Then, 3-chlorofuran (3.0 g; 29 mmol) and pyridine (1.6 mL) were added. The reaction mixture was stirred at 70°C during 22 h and was monitored by NMR analysis. The conversion is 95 % to an endo/exo mixture of Diels-Alder adducts.

The reaction mixture was concentrated in vacuo to give 2.27 g of a crude brown oil (containing 15% of residual pyridine) sufficiently pure for the next step.

### 1.2) Conversion of the Diels-Alder adducts previously obtained to 4,4'-dichlorodiphenylsulfone

In a Schlenk tube under argon atmosphere, powdered NaOH (90 mg, 2.2 mmol) is weighted and a solution of the previously obtained crude brown oil (511 mg) in DMSO (2.25 mL) is added. The reaction mixture was stirred at 40°C during 1 h. The reaction was monitored by NMR and GC analysis which shows the complete conversion of Diels-Alder adducts to dichlorodiphenylsulfones after 1 h. Gaz chromatography titration shows that the final mixture contains 184 mg of 4,4'-dichlorodiphenylsulfone and 43 mg of 3,4'-dichlorodiphenylsulfone, corresponding to a global yield over two steps for these two products of 59% and a 4,4'-dichlorodiphenylsulfone / 3,4'-dichlorodiphenylsulfone ratio of 81/19.

**1.3)** The crude brown oil obtained at step 1.1) was introduced in toluene in order to isolate the compound of formula (I), wherein X is chlorine. The recovered compound was characterized by the following NMR data: ¹H NMR (400 MHz, DMSO-d₆) δ 6.33 (dd J=2.0, 10.0 Hz, 2H), 5.36 (m, 2H), 5.03 (m, 2H), 4.10 (m, 2H), 2.36 (m, 2H), 1.60 (dt J=12.0, 4.4 Hz, 2H).

### 2) Preparation of 4,4'-dibromodiphenylsulfone

### 2.1) Diels-Alder reaction of 3-bromofuran with divinylsulfone

In a carousel tube fitted with a PTFE septum screw cap divinylsulfone (1.0 g, 8.46 mmol) was weighted. Then, 3-bromofuran (6.3 g, 42.9 mmol) and pyridine (3 mL) were added. The reaction mixture was stirred at 80°C during 24 h and was monitored by NMR analysis. The conversion is 95 % to an endo/exo mixture of Diels-Alder adducts.

The reaction mixture was concentrated in vacuo and purified by flash chromatography on silica gel (eluent: cyclohexane/ethyl acetate) to give a near quantitative yield (3.48 g) of a brown oil.

### 2.2) Conversion of the Diels-Alder adducts previously obtained to 4,4'-dibromodiphenylsulfone

In a Schlenk tube under argon atmosphere, potassium hydroxide, ca. 85% (35 mg, 0.53 mmol) is weighted and a solution of the previously obtained brown oil (215 mg, 0.52 mmol) in DMSO (1.5 mL) is added. The reaction mixture was stirred at 100°C during 2.5 h. The reaction was monitored by NMR and GC analysis which shows the complete conversion of Diels-Alder adducts to dibromodiphenylsulfone after 1 h.

Gaz chromatography titration shows that the final mixture contains 52 mg of 4,4'-dichlorodiphenylsulfone and 14 mg of 3,4'-dichlorodiphenylsulfone, giving a yield of these two products of 34% in this second step and a 4,4'-dibromodiphenylsulfone / 3,4'- dibromodiphenylsulfone ratio of 79/21.

**2.3)** The brown oil obtained at step 2.1) has been introduced in toluene in order to isolate the compound of formula (I), wherein X is bromine. The recovered compound is characterized by the following NMR data: ¹H NMR (400 MHz, DMSO-d₆) δ 6.50 (dd J=1.6, 10.0 Hz, 2H), 5.32 (m, 2H), 5.06 (m, 2H), 4.04 (m, 2H), 2.32 (m, 2H), 1.54 (m, 2H).

### 3) Preparation of a first polyarylethersulfone (polysulfone)

In a 250 ml. flask equipped with a stirrer, thermometer, a water cooled condenser and a Dean Stark moisture trap filled with benzene, there is placed 11.42 grams of 2,2-bis-(4-hydroxyphenyl)propane (0.05 moles), 13.1 grams of a 42.8% potassium hydroxide solution (0.1 moles KOH), 50 ml. of dimethylsulfoxide and 6 ml. benzene, and the system is purged with nitrogen to maintain an inert atmosphere over the reaction mixture. The mixture is refluxed for 3 to 4 hours, continuously removing the water contained in the reaction mixture as an azeotrope with benzene and distilling off enough of the latter to give a refluxing mixture at 130-135°C, consisting of the dipotassium salt of the 2,2-bis(4-hydroxyphenyl)propane and dimethylsulfoxide essentially free of water.

The mixture is cooled and 14.35 g (0.05 mole) of 4,4'-dichlorodiphenylsulfone is added followed by 40 ml. of anhydrous dimethylsulfoxide, all under nitrogen pressure. The mixture is heated to 130°C and held at 130-140°C with good stirring for 4-5 hours. The viscous, orange solution is poured into 300 ml. water, rapidly circulating in a Waring Blender, and the finely divided white polymer is filtered and then dried in a vacuum oven at 110°C for 16 hours. A substantial portion of the polymer is dissolved in 100 ml. tetrachloroethane and washed with dilute acetic acid and then water. After reprecipitation by pouring into 400 ml. of methanol, the polymer is filtered and dried in the vacuum oven at 70°C.

### 4) Preparation of a second polyarylethersulfone (polyethersulfone)

Bisphenol S (12.7g, 0.05mole), 4,4'-dichlorodiphenylsulfone (14.36 g, 0.05 mole) and anhydrous potassium carbonate (6.91 g, 0.05 mole) are charged to a 3-necked flask (capacity 100 cm³) fitted with a stirrer, air condenser and nitrogen inlet. The mixture is heated on an oil bath to 200°C under a nitrogen stream without stirring to effect melting. When the temperature reaches 200°C the stirrer is started and the mixture is stirred at 200°C for two hours. The temperature is raised to 260°C when some frothing occurs and the mixture becomes more viscous. After 31/2 hours at 260°C the temperature is increased to 280°C when more frothing occurs. After 1/2 hour at 280°C the temperature is raised to 296°C whereat the frothing gradually subsides and the mixture becomes slowly more viscous. After a further 6 hours at 290°C the mixture is too viscous to stir and is left unstirred at 290°C for a further 3 hours. Throughout the reaction the mixture remains a pale off-white colour. The mixture is then cooled and the polymer isolated by dissolution in dimethyl formamide and precipitation. The polymer is washed with water and dried to give a white powder.

### 5) Preparation of a third polyarylethersulfone (polyphenylsulfone)

A 500 mL, 4-neck round bottom flask is equipped through its center neck with an overhead stirrer attached to a stainless steel paddle. Through one of its side necks, a Claisen adapter is attached. A thermocouple thermometer is inserted through the Claisen adapter which is in-turn attached to a temperature controller. The other neck of the Claisen is attached to a Dean-Stark trap and a water cooled condenser. A gas inlet tube and a stopper are placed in the other necks of the round bottom flask. The reactor is placed in an oil bath which is connected to the temperature controller.

4,4'-biphenol (26.068 g), 4,4'-dichlorodiphenylsulfone (40.205 g), anhydrous potassium carbonate (20.509 g, 6% excess), sulfolane (68.4 g) and chlorobenzene (43.6 g) are charged to the reactor. The agitator is started to 300 rpm. The whole reactor is evacuated with pump vacuum and filled with nitrogen. The degassing operation is accomplished two more times. A steady stream of nitrogen through the reactor solution is started. The reactor temperature is set to 220°C and the stirring speed is increased to 400 rpm. Care is taken not to splash the reaction solution too high on the reaction flask walls. The chlorobenzene which distills, along with the water of the reaction that is formed, is collected in the Dean-Stark trap and not returned to the reaction flask. Thus, the chlorobenzene initially added and the water formed is removed from the reaction mixture as the temperature of the reaction mixture increases. The desired temperature is reached in about 30 to about 40 minutes. The reactor temperature is maintained at 220 °C. When the viscosity starts to increase the agitator speed is increased to 500 rpm. At the time selected to end the polymerization reaction, generally after 70 to 80 minutes of reaction time after reaction reaches 220°C (at this stage substantially all of the chlorobenzene is distilled out of the reaction mixture), a mixture of sulfolane (90 g) and chlorbenzene (50 g) is slowly added from an addition funnel to cool the reaction. The temperature controller is reset to 160°C. The distilled chlorobenzene is either removed from the reaction mixture or returned to maintain the temperature of 160°C. Methyl chloride gas is added for 30 minutes (18 to 22 g). Shortly after the addition of methyl chloride is started, an aqueous solution of potassium carbonate (0.4 g in 3 ml water) is added via syringe. After 30 minutes of methyl chloride addition, the oil bath is removed. The reactor solution is diluted with 200 ml chlorobenzene to allow filtration. To remove salts, the polymer solution is filtered through a 2 µm filter in a pressure filter funnel using 10-20 psig nitrogen. The polymer is recovered by slowly adding the salt-free solution (up to 100 mL) into a mixture of 500 g of methanol and water in a ratio of 70:30 under high speed agitation in a blender with a cover containing a small opening through which the polymer solution is added. The precipitate is recovered by filtration and returned to the blender. Successive washings of the precipitate in the blender are completed using 400 g methanol, 400 g deionized water and finally with 400 g methanol. The washed precipitate is filtered one more time and dried in a vacuum oven (60 mm) at 120°C.

### 6) Preparation of 4,4'-diaminodiphenylsulfone

Into the copper liner of an Adkins rocking bomb is placed 20 g. of 4,4'-dichlorodiphenyl sulfone, 1 g. of cuprous bromide, 0.5 g. copper bronze and 170 cc. of 28% aqueous ammonia. The bomb is shaken at 200° for about 40 hours and shaking is continued during the cooling period to avoid solidification of the product as a solid cake. The solid is filtered from the ammoniacal liquid, washed with water, and dissolved in 10% hydrochloric acid. A small amount of tar and the copper bronze are removed by treatment with Darco® activated carbon and filtration. The solution is cooled and the clear brownish liquid is basified with ammonia. The collected precipitate is washed with ammonium hydroxide and water and dissolved in 150 cc. of ethanol and 50 cc. of water. A voluminous precipitate of ferric hydroxide is left undissolved, and it is filtered with addition of standard Super Cel™ calcined diatomaceous earth filter aid. The slightly brownish filtrate is clarified with Darco® activated carbon and diluted with boiling water to a volume of about 800 cc. Flat needles of 4,4'-diaminodiphenylsulfone crystallize on cooling.

## Claims

1. Compound of formula (I) 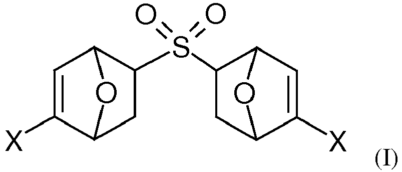 wherein X represents a halogen atom selected from the group consisting of fluorine, chlorine, bromine and iodine.

2. Compound according to claim 1, wherein the halogen atom is chlorine or bromine, preferably chlorine.

3. Process for the preparation of a compound of formula (I) as defined in claim 1 or 2, comprising reacting divinylsulfone with a halofuran of formula (II) 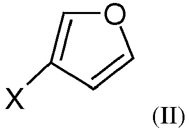 wherein X is as defined above,
and optionally further isolating compound of formula (I).

4. Process according to claim 3, wherein the halofuran is 3-chlorofuran or 3-bromofuran.

5. Process according to claim 3 or 4, wherein the molar ratio of the halofuran of formula (II) to divinylsulfone is of at least 2/1.

6. Use of a compound of formula (I) as defined in claim 1 or 2, for the preparation of a 4,4'-dihalodiphenylsulfone of formula (III) 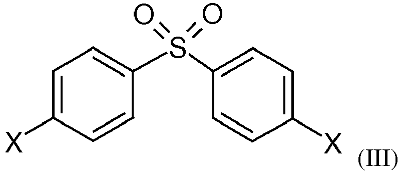 wherein X is as defined above.

7. Process for the preparation of a 4,4'-dihalodiphenylsulfone of formula (III) 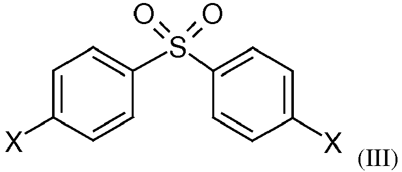 wherein X represents a halogen atom selected from the group consisting of fluorine, chlorine, bromine and iodine,
comprising carrying out the dehydration/aromatization of a compound of formula (I) 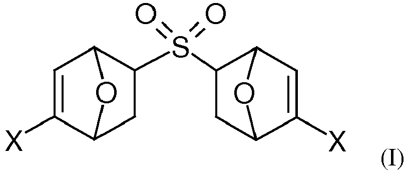 wherein X is as defined above.

8. Process according to claim 7, wherein the dehydration/aromatization is carried out in the presence of an alkali hydroxide, preferably sodium hydroxide or potassium hydroxide.

9. Process according to claim 7 or 8, wherein the dehydration/aromatization is carried out in the presence of DMSO.

10. Process for the preparation of a 4,4'-dihalodiphenylsulfone of formula (III) 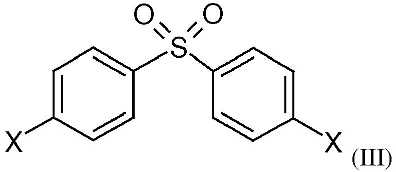 wherein X represents a halogen atom selected from the group consisting of fluorine, chlorine, bromine and iodine,
comprising:
a) providing a compound of formula (I) 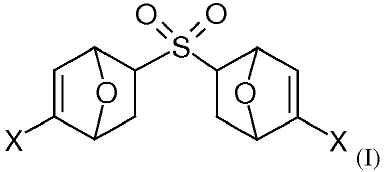 wherein X is as defined above,
b) carrying out the dehydration/aromatization of said compound of formula (I),
c) optionally further isolating compound of formula (III).

11. Process according to claim 10, wherein compound of formula (I) is prepared by reacting divinylsulfone with a halofuran of formula (II) 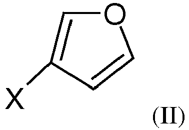 wherein X is as defined above.

12. Use of the 4,4'-dihalodiphenylsulfone prepared by the process according to any one of claims 7 to 11 for the manufacture of a polyarylethersulfone, such as a polysulfone, a polyethersulfone or a polyphenylsulfone.

13. Process for the manufacture of a polyarylethersulfone comprising the steps of :
i) preparing a 4,4'-dihalodiphenylsulfone of formula (III) 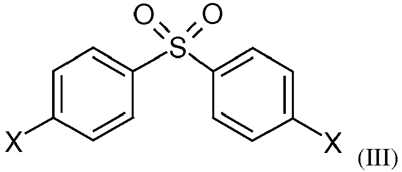 wherein X represents a halogen atom selected from the group consisting of fluorine, chlorine, bromine and iodine,
by the process according to any one of claims 7 to 11,
ii) optionally, isolating the 4,4'-dihalodiphenylsulfone,
iii) forming a reaction mixture comprising the 4,4'-dihalodiphenylsulfone and at least one of M¹ and M², wherein M¹ is at least one dibasic salt of a dihydroxy aromatic compound and M² consists of at least one dihydroxy aromatic compound and at least one basic compound,
iv) carrying out a polycondensation reaction of the reaction mixture.

14. Process for the manufacture of 4,4'-diaminodiphenylsulfone comprising the steps of :
i) preparing a 4,4'-dihalodiphenylsulfone of formula (III) 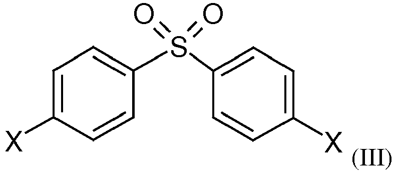 wherein X represents a halogen atom selected from the group consisting of fluorine, chlorine, bromine and iodine,
by the process according to any one of claims 7 to 11,
ii) optionally, isolating the 4,4'-dihalodiphenylsulfone,
iii) carrying out the amination reaction of the 4,4'-dihalodiphenylsulfone with an aminating agent, especially carrying out the ammonolysis reaction of the 4,4'-dihalodiphenylsulfone with ammonia.

15. Process according to claim 13 or 14, which comprises the step ii) of isolating the 4,4'-dihalodiphenylsulfone.

## Patentansprüche

1. Verbindung der Formel (I) 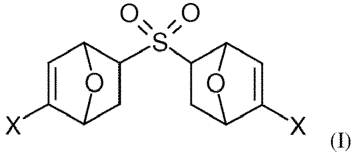 wobei X für ein Halogenatom aus der Gruppe bestehend aus Fluor, Chlor, Brom und Iod steht.

2. Verbindung nach Anspruch 1, wobei es sich bei dem Halogenatom um Chlor oder Brom, vorzugsweise Chlor, handelt.

3. Verfahren zur Herstellung einer Verbindung der Formel (I) gemäß Anspruch 1 oder 2, bei dem man Divinylsulfon mit einem Halogenfuran der Formel (II) 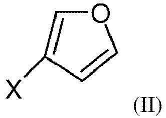 wobei X wie oben definiert ist,
umsetzt und gegebenenfalls ferner die Verbindung der Formel (I) isoliert.

4. Verfahren nach Anspruch 3, wobei es sich bei dem Halogenfuran um 3-Chlorfuran oder 3-Bromfuran handelt.

5. Verfahren nach Anspruch 3 oder 4, wobei das Molverhältnis von Halogenfuran der Formel (II) zu Divinylsulfon mindestens 2/1 beträgt.

6. Verwendung einer Verbindung der Formel (I) gemäß Anspruch 1 oder 2 zur Herstellung eines 4,4'-Dihalogendiphenylsulfons der Formel (III) 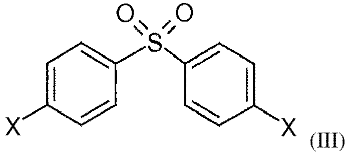 wobei X wie oben definiert ist.

7. Verfahren zur Herstellung eines 4,4'-Dihalogendiphenylsulfons der Formel (III) 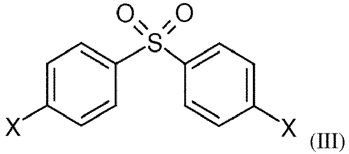 wobei X für ein Halogenatom aus der Gruppe bestehend aus Fluor, Chlor, Brom und Iod steht, bei dem man die Dehydratisierung/Aromatisierung einer Verbindung der Formel (I) 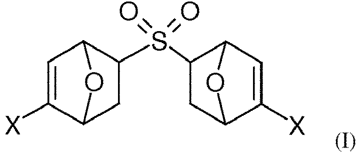 wobei X wie oben definiert ist,
durchführt.

8. Verfahren nach Anspruch 7, wobei man die Dehydratisierung/Aromatisierung in Gegenwart von einem Alkalihydroxid, vorzugsweise Natriumhydroxid oder Kaliumhydroxid, durchführt.

9. Verfahren nach Anspruch 7 oder 8, wobei man die Dehydratisierung/Aromatisierung in Gegenwart von DMSO durchführt.

10. Verfahren zur Herstellung eines 4,4'-Dihalogendiphenylsulfons der Formel (III) 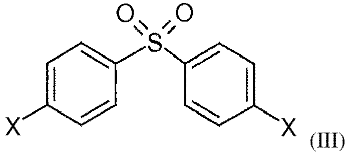 wobei X für ein Halogenatom aus der Gruppe bestehend aus Fluor, Chlor, Brom und Iod steht, bei dem man
a) eine Verbindung der Formel (I) 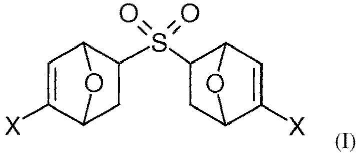 wobei X wie oben definiert ist,
bereitstellt,
b) die Dehydratisierung/Aromatisierung der Verbindung der Formel (I) durchführt,
c) gegebenenfalls ferner die Verbindung der Formel (III) isoliert.

11. Verfahren nach Anspruch 10, wobei die Verbindung der Formel (I) durch Umsetzen von Divinylsulfon mit einem Halogenfuran der Formel (II) 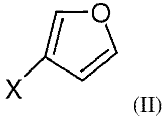 wobei X wie oben definiert ist,
hergestellt wird.

12. Verwendung des durch das Verfahren nach einem der Ansprüche 7 bis 11 hergestellten 4,4'-Dihalogendiphenylsulfons zur Herstellung eines Polyarylethersulfons, wie eines Polysulfons, eines Polyethersulfons oder eines Polyphenylsulfons.

13. Verfahren zur Herstellung eines Polyarylethersulfons, das folgende Schritte umfasst:
i) Herstellen eines 4,4'-Dihalogendiphenylsulfons der Formel (III) 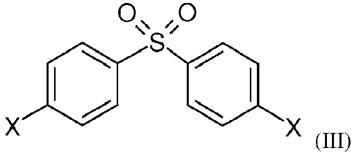 wobei X für ein Halogenatom aus der Gruppe bestehend aus Fluor, Chlor, Brom und Iod steht, durch das Verfahren nach einem der Ansprüche 7 bis 11,
ii) gegebenenfalls Isolieren des 4,4'-Dihalogendiphenylsulfons,
iii) Bilden einer Reaktionsmischung, die das 4,4'-Dihalogendiphenylsulfon und mindestens eines von M¹ und M² umfasst, wobei M¹ mindestens ein zweibasiges Salz einer aromatischen Dihydroxyverbindung ist und M² aus mindestens einer aromatischen Dihydroxyverbindung und mindestens einer basischen Verbindung besteht,
iv) Durchführen einer Polykondensationsreaktion der Reaktionsmischung.

14. Verfahren zur Herstellung von 4,4'-Diaminodiphenylsulfon, das folgende Schritte umfasst:
i) Herstellen eines 4,4'-Dihalogendiphenylsulfons der Formel (III) 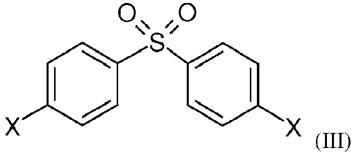 wobei X für ein Halogenatom aus der Gruppe bestehend aus Fluor, Chlor, Brom und Iod steht, durch das Verfahren nach einem der Ansprüche 7 bis 11,
ii) gegebenenfalls Isolieren des 4,4'-Dihalogendiphenylsulfons,
iii) Durchführen der Aminierungsreaktion des 4,4'-Dihalogendiphenylsulfons mit einem Aminierungsmittel, insbesondere Durchführung der Ammonolysereaktion des 4,4'-Dihalogendiphenylsulfons mit Ammoniak.

15. Verfahren nach Anspruch 13 oder 14, das den Schritt ii) des Isolierens des 4,4'-Dihalogendiphenylsulfons umfasst.

## Revendications

1. Composé de formule (I) 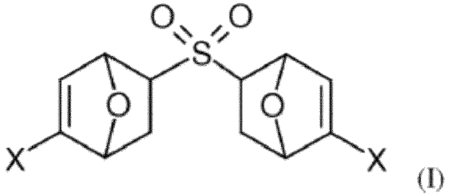 X représentant un atome d'halogène choisi dans le groupe constitué par fluor, chlore, brome et iode.

2. Composé selon la revendication 1, l'atome d'halogène étant le chlore ou le brome, préférablement le chlore.

3. Procédé pour la préparation d'un composé de formule (I) tel que défini dans la revendication 1 ou 2, comprenant la mise en réaction de divinylsulfone avec un halogénofuranne de formule (II) 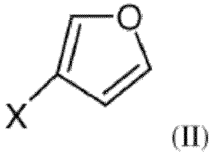 X étant tel que défini ci-dessus,
et éventuellement l'isolement ultérieur d'un composé de formule (I).

4. Procédé selon la revendication 3, l'halogénofuranne étant le 3-chlorofuranne ou le 3-bromofuranne.

5. Procédé selon la revendication 3 ou 4, le rapport molaire de l'halogénofuranne de formule (II) à la divinylsulfone étant d'au moins 2/1.

6. Utilisation d'un composé de formule (I) tel que défini dans la revendication 1 ou 2, pour la préparation d'une 4,4'-dihalogénodiphénylsulfone de formule (III) 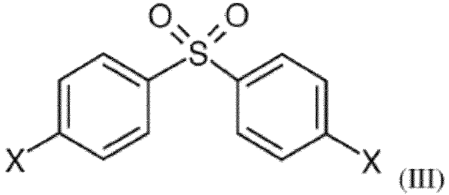 X étant tel que défini ci-dessus.

7. Procédé pour la préparation d'une 4,4'-dihalogénodiphénylsulfone de formule (III) 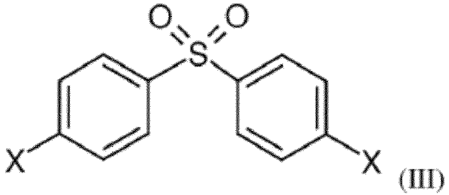 X représentant un atome d'halogène choisi dans le groupe constitué par le fluor, le chlore, le brome et l'iode,
comprenant la mise en œuvre de la déshydratation/aromatisation d'un composé de formule (I) 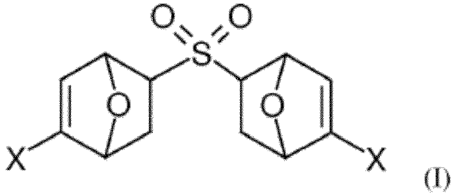 X étant tel que défini ci-dessus.

8. Procédé selon la revendication 7, la déshydratation/aromatisation étant mise en œuvre en présence d'un hydroxyde d'alcali, préférablement d'hydroxyde de sodium ou d'hydroxyde de potassium.

9. Procédé selon la revendication 7 ou 8, la déshydratation/aromatisation étant mise en œuvre en présence de DMSO.

10. Procédé pour la préparation d'une 4,4'-dihalogénodiphénylsulfone de formule (III) 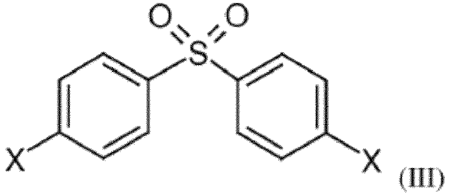 X représentant un atome d'halogène choisi dans le groupe constitué par le fluor, le chlore, le brome et l'iode,
comprenant :
a) la mise à disposition d'un composé de formule (I) 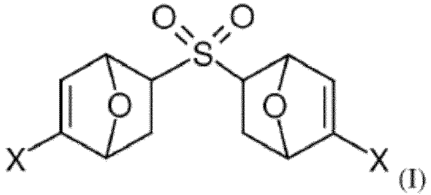 X étant tel que défini ci-dessus,
b) la mise en œuvre de la déshydratation/aromatisation dudit composé de formule (I),
c) éventuellement l'isolement ultérieur d'un composé de formule (III).

11. Procédé selon la revendication 10, un composé de formule (I) étant préparé par la mise en réaction de divinylsulfone avec un halogénofuranne de formule (II) 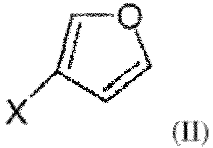 X étant tel que défini ci-dessus.

12. Utilisation de la 4,4'-dihalogénodiphénylsulfone préparée par le procédé selon l'une quelconque des revendications 7 à 11 pour la fabrication d'une polyaryléthersulfone, telle qu'une polysulfone, une polyéthersulfone ou une polyphénylsulfone.

13. Procédé pour la fabrication d'une polyaryléthersulfone comprenant les étapes de :
i) préparation d'une 4,4'-dihalogénodiphénylsulfone de formule (III) 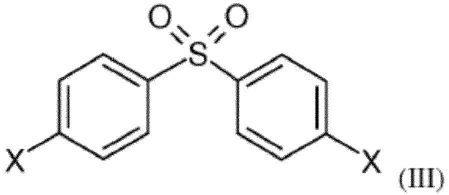 X représentant un atome d'halogène choisi dans le groupe constitué par le fluor, le chlore, le brome et l'iode,
par le procédé selon l'une quelconque des revendications 7 à 11,
ii) éventuellement, l'isolement de la 4,4'-dihalogénodiphénylsulfone,
iii) la formation d'un mélange réactionnel comprenant la 4,4'-dihalogénodiphénylsulfone et au moins l'un parmi M¹ et M², M¹ étant au moins un sel dibasique d'un composé dihydroxyaromatique et M² étant constitué d'au moins un composé dihydroxyaromatique et au moins un composé basique,
iv) la mise en œuvre d'une réaction de polycondensation du mélange réactionnel.

14. Procédé pour la fabrication d'une 4,4'-diaminodiphénylsulfone comprenant les étapes de :
i) préparation d'une 4,4'-dihalogénodiphénylsulfone de formule (III) 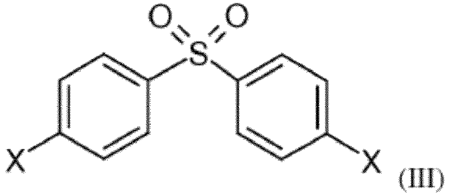 X représentant un atome d'halogène choisi dans le groupe constitué par le fluor, le chlore, le brome et l'iode,
par le procédé selon l'une quelconque des revendications 7 à 11,
ii) éventuellement, l'isolement de la 4,4'-dihalogénodiphénylsulfone,
iii) la mise en œuvre de la réaction d'amination de la 4, 4'-dihalogénodiphénylsulfone avec un agent d'amination, notamment la mise en œuvre de la réaction d'ammonolyse de la 4,4'-dihalogénodiphénylsulfone avec de l'ammoniac.

15. Procédé selon la revendication 13 ou 14, qui comprend l'étape ii) d'isolement de la 4,4'-dihalogénodiphénylsulfone.
